# EUROPÄISCHE PATENTSCHRIFT

(11) **EP 0 476 574 B1**
(45) Veröffentlichungstag und Bekanntmachung des Hinweises auf die Patenterteilung: **23.05.2001**
(21) Anmeldenummer: 91115708.9
(22) Anmeldetag: 17.09.1991
(51) Int. Cl.: B01J 20/26, A61L 15/00

(54) **Verfahren zur Herstellung von Wasseradsorptionsmaterial und dessen Verwendung in Hygieneartikeln und zur Bodenverbesserung**
Process for manufacturing water adsorbing material and its use in sanitary articles and in soil amelioration
Procédé pour la fabrication de matériau adsorbant l'eau et son utilisation dans les articles hygièniques et dans l'amélioration des sols

(30) Priorität: 19.09.1990 DE 4029593
(43) Veröffentlichungstag der Anmeldung: 25.03.1992
(60) Teilanmeldung: 00117777.3
(73) Patentinhaber: Stockhausen GmbH & Co. KG, 47805 Krefeld (DE)
(72) Erfinder: Chmelir, Miroslav, Dr., W-4150 Krefeld (DE)
(74) Vertreter: Wolff, Felix, Dr.

(56) Entgegenhaltungen:
- EP-A- 0 071 063
- EP-A- 0 181 983
- WO-A-90/09236
- DE-A- 2 143 549
- DE-A- 2 264 027

## Beschreibung

Die Erfindung betrifft ein Verfahren zur Herstellung von Absorptionsmaterialien, die aus einer Kombination von synthetischen Polymeren und Stärke Stärkederivaten, modifizierte Stärke, bestehen, die Wasser und wässrige Flüssigkeiten schnell aufnehmen und die z.B. für absorbierende Wegwerferzeugnisse in Hygieneartikeln, wie Windeln und Damenbinden, und für andere medizinische Zwecke oder auch als wasserspeichernde Bodenverbesserungsmittel verwendet werden.

Absorptionsmittel mit hohem Absorptionsvermögen für Wasser und Körperflüssigkeiten sind bekannt. Zu den vollsynthetischen Absorptionsmitteln gehören vernetzte Polymere und Copolymere auf Acryl- oder Methacrylsäurebasis (DE-OS 24 29 236, DE-OS 26 14 662, US-PS 4 018 951, US-PS 3 926 891, US-PS 4 066 583, US-PS 4 062 817, DE-OS 27 12 043, DE-OS 26 53 135, DE-OS 26 50 377, DE-OS 28 13 634), Maleinsäurederivate (nach US-PS 4 041 228) oder Acrylamidopropansulfonsäurecopolymerisate (nach DE-PS 31 24 008). Diese bekannten synthetischen Absorptionsmittel sind praktisch wasserunlöslich, absorbieren im Gleichgewicht das Vielfache ihres Gewichts an Wasser, Urin oder anderen wässrigen Lösungen, sind jedoch relativ beständig gegen biologische Abbaubarkeit.

Weitere Produkte wurden auf Stärkebasis, wie z.B. Stärke-Acrylnitril-Pfropfpolymerisate (nach US-PS 3 997 484, US-PS 3 661 815, US-PS 4 155 888, US-PS 3 935 099), gelatinisierte Stärkederivate (nach DE-OS 27 02 781) oder auf Cellulosebasis, wie Derivate von Alkyl- oder Hydroxyalkylcellulose (nach JP-PS 77/125 481), Carboxymethylcellulose (nach BE-PS 862 130, GB-PS 1 159 949) und auf Polysaccharidbasis (nach DE-OS 26 50 377) hergestellt. Diese Produkte, wie die mit Acrylnitril- oder Acrylsäure gepfropften Stärkepolymerisate gehören, zwar zu den abbaubaren Produkten, jedoch ist ihre Herstellung sehr aufwendig und die Menge des Naturprodukts im Endprodukt ist durch die hohe Viskosität des Reaktionsmediums, wie dies z.B. bei einer Monomerlösung mit gelöster Stärke der Fall ist, stark begrenzt. Beispielsweise enthält die Monomerenlösung nach EP 0 372 981 Beispiel 11 nur ca. 4 Gew% gelöste Stärke, bezogen auf den gesamten Ansatz.

Stärkeprodukte, Dextrin oder Mehl werden gemäß DE-PS 21 43 549 auch zur Bepuderung der Gallert-Granulate von Polyacrylamidpolymerisaten benutzt, um lagerfähige, nicht zusammenbackende, formstabile Gallert-Granulate mit hohem Wassergehalt zu erhalten, deren Verwendung als wasserlösliche Flockungs- und Sedimentationshilfsmittel vorgeschlagen wird.

In diesem Fall ist die Stärke nur auf der Oberfläche der wasserhaltigen Polymergelgranulate eines wasserlöslichen Produkts verteilt. Beim ersten Kontakt mit Wasser wird die Stärke von der Oberfläche des bepuderten Polymergels weitgehend abgespült. Eine Einbindung der Stärke in den Polyacrylamidkörper ist hier nicht vorhanden.

So ist ein Verfahren, das Naturprodukt (im weiteren als Komponente B bezeichnet) direkt zu der Monomerlösung der Komponente A (synthetisches Polymerisat) zuzusetzen, zu homogenisieren und danach die Polymerisation zu starten, nur bei niedrigen Gehalten von 2 bis höchstens 5 Gew.-% an Komponente B in der Monomerlösung durchführbar, während aus technischen und wirtschaftlichen Gründen die Monomerkonzentration der Monomerlösung zwischen 20 - 30 Gew.% liegt. Solche Verfahren sind z. B. in den (DE-PS 26 12 846) GB 1490128 und EP-PS 0 189 163 beschrieben. Die Versuche, höhere Mengen als 5 Gew.-% an Komponente B in der Monomerlösung zu lösen, führen zu hochviskosen bis gelartigen, unrührbaren Monomerlösungen, die keine homogene Vermischung mit den Katalysatorlösungen erlauben, so daß eine gleichmäßige, bis zu hohem Umsatz geführte Polymerisation unmöglich ist.

Auch inerte Füllmittel wie Holzmehl, Zellstoffasern, Zellulose- oder Nylonflocken, Schlacke, Ton, Flugasche, Kohlenstaub und Düngemittel werden gemäß DE-AS 22 64 027 als Zusätze bei der Vernetzung der wasserlöslichen Polymerisate verwendet. Die nichtvernetzten wasserlöslichen Polymerisate bzw. Copolymerisate werden als getrocknetes Pulver mit den inerten Füllmitteln vermischt, und nach einer Besprühung des Gemisches mit 15 - 85 Gew.-% oder sogar noch mehr Wasser (in den meisten Beispielen werden 200 - 400 Gew.-% Wasser, bezogen auf das Gemisch Polymerisat und Füllmittel, verwendet) wird das entstandene wasserhaltige Granulat durch ionisierende Bestrahlung (0,05 - 20,0 MeV) vernetzt. Nach der Trocknung entsteht ein z. T. wasserunlösliches polymeres Absorptionsmaterial, dessen Wasserabsorptionskapazität etwa das 5 - 40fache des Trockengewichtes beträgt.

WO-A-9009236 beschreibt ein Verfahren zur Herstellung von Absorptionsmaterial für Wasser aus wenigstens zwei Komponenten A und B, nämlich einem wasserquellbaren, synthetischen Polymeren (A) und einer polymeren Verbindung (B), wobei die Komponente (B) in trockener und teilweise gequollener Form der Komponente (A) während ihres Herstellungsprozesses zugesetzt wird. Aufgabe der WO-A-9009236 ist es, ein universell geeignetes Bindemittel für Flüssigkeiten bereitzustellen, das die Nachteile bekannter Bindemittel nicht auf weist. Einen Hinweis auf die Eignung als Absorptionsmittel mit einem hohen Aufnahmevermögen für wäßrige Flüssigkeiten wie Urin oder Blut findet sich nicht.

EP-A-0 071 063 beschreibt ein Absorptionsmittel für Blut und seröse Körperflüssigkeiten, das aus wenigsten zwei Komponenten A und B besteht, wobei die Komponente A ein vernetztes, wasserquellbares, synthetische oder natürliches Polymeres oder Copolymeres und die Komponente B eine anorganische und/oder organische, bei normaler Temperatur feste wasserlösliche Verbindung ist. Durch den Zusatz der wasserlöslichen Komponenten B zur Komponente A soll das kapillare Fließen des Blutes durch die Masse des teilchenförmigen Absorptionsmittel beschleunigt werden, um eine schnellere Verteilung des Blutes im Absorptionsmittel und somit eine schnellere Blutabsorption zu ermöglichen.

Aufgabe der Erfindung ist die Bereitstellung eines Verfahrens, das es ermöglicht, die bekannten, als Absorptionsmittel eingesetzten synthetischen Polymerisate mit hohem Aufnahmevermögen für Wasser und wäßrige Flüssigkeiten wie Urin oder Blut durch Zusätze, die selbst ein vergleichsweise geringes Aufnahmevermögen für derartige Flüssigkeiten haben, einzubinden, ohne daß sich das Absorptionsvermögen des Verfahrensproduktes auch bei Einbindung von höheren Mengen an schlechter absorbierenden Zusätzen nennenswert verschlechtert. Gleichzeitig wird in bestimmten Fällen noch die Abbaubarkeit verbessert.

Diese Aufgabe wird erfindungsgemäß durch die kennzeichnenden Merkmale von Patentanspruch 1 gelöst.

Die Herstellung der Komponente A erfolgt auf bekannte Weise. So kann die Herstellung der Komponente A als gequollenes Polymergel diskontinuierlich in einem Polymerisationsgefäß oder kontinuierlich auf einem endlosen Band erfolgen. Gemäß DE-PS 35 44 770 wird z.B. die Polymerisation in einer wässrigen Lösung, enthaltend das wasserlösliche Monomere und ggf. die Comonomeren, in einer Konzentration von 2,2 bis 8,3 Mole polymerisationsfähiger Doppelbindungen pro Kilo Monomerenlösung, insbesondere 3,5 bis 6,25 Mole (entsprechend 16 - 60 Gew.-%,insbesondere 25 - 45 Gew.-% Acrylsäure, wenn diese als Monomere verwendet wird) in einem Temperaturbereich von etwa - 10 bis 120°C durchgeführt.

Als Komponente A kommen in erster Linie die Polymerisate von Acrylsäure und Methacrylsäure, alleine als Homopolymerisat oder als Copolymerisat, ferner aber auch die Polymerisate von anderen wasserlöslichen Monomeren wie Acrylamid sowie polymerisationsfähige Säuren und ihre Salze, insbesondere Malein-, Fumar-, Itacon-, Vinylsulfon- oder 2-Acrylamido-2-methylpropansulfonsäure in Frage. Insbesondere kommt als Komponente A ein vernetztes Polymeres oder Copolymeres auf Basis von Acrylsäure, Methacrylsäure, Derivaten dieser Carbonsäuren, vorzugsweise ein Homo- oder Copolymerisat der Acryl-, Methacryl-, 2-Acrylamido-2-methylpropansulfonsäure, den Alkali- oder Ammoniumsalzen dieser Carbonsäuren, des Acryl- oder Methacrylamids und deren Derivaten, des Vinylpyrrolidons sowie deren Copolymerisaten untereinander oder mit anderen nur teilweise wasserlöslichen Monomeren zum Einsatz Ferner können hydroxygruppenhaltige Ester polymerisationsfähiger Säuren, insbesondere die Hydroxyethyl- und Hydroxypropylester der Acryl- und der Methacrylsäure verwendet werden, ebenso aminogruppenhaltige und ammoniumgruppenhaltige Ester und Amide polymerisationsfähiger Säuren, wie die Dialkylaminoester, insbesondere die Dimethyl- und die Diethylaminoalkylester der Acryl- und der Methacrylsäure sowie die Trimethyl- und die Triethylammoniumalkylester und die entsprechenden Amide. Weiterhin werden in geringen Anteilen vernetzende Monomere, wie z.B. Monomere mit mehr als einer polymerisationsfähigen Gruppe im Molekül zusammen mit den vorstehenden Monomeren polymerisiert.

Die vorstehenden Monomeren können allein zu vernetzten Homo- oder untereinander zu vernetzten Copolymerisaten polymerisiert werden.

In geringen Mengen können noch in Wasser schwerlösliche oder sogar wasserunlösliche Monomere wie (Meth)acrylnitril, Vinylpyridin und Vinylacetat copolymerisiert werden wie die Ester der Acryl- und/oder Methacrylsäure mit C₁-C₁₀-Alkoholen, Styrol und alkylierte Styrole. Im allgemeinen liegt der Anteil an den wasserlöslichen Monomeren bei 40 bis 100 Gew.-%, bezogen auf die Gesamtheit der Monomeren. Der Anteil an den vernetzenden Monomeren liegt bei 0 bis 20 Gew.-%, vorzugsweise bei 0,01 bis 2,0 Gew-%, bezogen auf die Gesamtheit der Monomeren. Die wasserunlöslichen, hydrophoben Monomeren machen in der Regel 0 bis 40 Gew.-% der Monomeren aus.

Als vernetzende Monomere seien bi- oder mehrfunktionelle Monomere, z.B. Amide, wie das Methylenbisacryl- bzw. -methacrylamid oder Ethylenbisacrylamid, ferner Ester der ungesättigten Mono- oder Polycarbonsäuren von Polyolen, wie Diacrylate oder Triacrylate, z.B. Butandiol- oder Ethylenglykoldiacrylat bzw. -methacrylat, Trimethylolpropantriacrylat, ferner Vinylmethacrylat und Allylverbindungen wie Allyl(meth)acrylat, Triallylcyanurat, Maleinsäurediallylester, Polyallylester, Tetraallyloxiethan, Triallylamin, Tetraallylethylendiamin, Allylester der Phosphorsäure bzw. phosphorigen Säure, ferner vernetzungsfähige Monomere, wie die N-Methylolverbindungen von Amiden, wie Methacrylamid bzw. Acrylamid und die davon abgeleiteten Äther.

Die Polymerisation kann durch chemische Katalyse und/oder durch energiereiche Strahlung/Licht initiiert werden. Als geeignete Katalysatoren können z.B. Peroxiverbindungen wie Kaliumperoxidisulfat, Wasserstoffperoxid, organische Peroxide wie Benzoylperoxid, tert. Butylhydroperoxid, tert. Butylperpivalat, Redox-Systeme, wie z.B. Kaliumperoxidisulfat-Natriumdisulfit, Wasserstoffperoxid-Hydroxylaminchlorid oder Azoinitiatoren wie AIBN (2,2'-Azobis(isobutyronitril)) oder 2,2'-Azobis(2-amidinopropan)dihydrochlorid verwendet werden. Als Photoinitiatoren können z.B. Benzoin und seine Derivate, z.B. Benzoinether wie Benzoin-Ethyl-Propyl-Ether, Benzil und seine Derivate, wie Benzilketale oder Acryldiazoniumsalze, Acetophenonderivate und andere alleine oder in Gemischen. Im allgemeinen liegt der Gehalt an Photoinitiatoren bei 0,002 bis 2,0 Gew.-%, vorzugsweise 0,01 bis 0,2 Gew.-%, bezogen auf die eingesetzten Monomeren. Der Gehalt an Katalysatoren liegt im allgemeinen bei 0,02 bis 5,0 Gew.-%, vorzugsweise zwischen 0,20 bis 2,0 Gew.-%, bezogen auf die Monomeren.

Als Komponente B werden erfindungsgemäß Stärke und Stärkederivate, wie z.B. Mais-, Korn-, Kartoffelstärke, Amylose, Amylopektin, Dextrin, Dextran, modifizierte Stärke, Hydroxyethylstärke, kationische Stärke, Stärkepfropfpolymerisate verwendet.

Zusätzlich zur Komponente B können noch andere Materialien mit großer Oberfläche, wie z.B. Fasermaterials aus Naturfasern, bevorzugt Baumwolle-, Hanf-, Wolle- und Seidenfasern, weiterhin Fasermaterial aus Cellulosefasern wie Viskose-, Acetat- und Triacetatfasern oder aus synthetischen Fasern auf der Basis von Polyester, Polyolefinen, Polyacrylnitril, Polyamid, Polyvinylalkohol, -acetat und -chlorid, Polyurethan, Polyvinylharnstoff, Polyharnstoff und der Copolymerisate von diesen Polymeren, verwendet werden. Die Fasermaterialien können bevorzugt als Kurzschnittfaser von einer Länge von 0,1 bis 60 mm in die Komponente A eingearbeitet werden.

Weitere Materialien wie Riechstoffe zur Parfümierung des Endproduktes, Desinfektionsmittel, antibakterielle Mittel, aber auch neutrale Füllstoffe, wie z.B. Holzmehl, Torf, gemahlene Nußschalen wie Walnußschalen, Kernobstschalen, chitinhaltiges Mehl, Sand, Tonerde und/oder Erde für Pflanzenzucht, können im Gemisch, mit der komponente B verwendet werden.

Das erfindungsgemäße Verfahren besteht darin, daß die Komponente B als Pulver in trockenem bzw. in leicht angefeuchtetem bis gequollenem Zustand während der Herstellung des synthetischen Polymeren (Komponente A) zu dem gequollen Polymergel der Komponente A zugesetzt wird. Vorteilhaft ist, wenn die Komponente B erst in der Endphase der Herstellung der Komponente A, d.h. erst nachdem ein Umsatz von mehr als 30 %, bevorzugt mehr als 60 % und besonders bevorzugt mehr als 95 %, erreicht wird, zu dem gequollenen Polymergel der Komponente A zugesetzt, mit dem Polymergel vermischt und anschließend getrocknet wird.

Die Vermischung der beiden Komponenten kann in einer geeigneten Mischeinrichtung erfolgen. Vorteilhaft wird hierzu ein Mischer mit rotierendem Mischwerkzeug verwendet. Ein geeigneter Mischer besteht z.B. aus einem vertikal oder horizontal gelagerten Metallzylinder, dessen Mischwerkzeug mit Leitschaufeln, die die Wände des Mischerzylinders gründich abstreifen, ausgerüstet ist.

Für eine gleichmäßige Durchmischung der beiden Komponenten A und B kann auch ein Trogkneter mit Doppel-U-Querschnitt verwendet werden. Im Trog bewegt sich ein Knetwellenpaar gleich- oder gegensinnig, gleich oder verschieden schnell, wobei auch die Schaufelform je nach Mischgut gezielt gewählt werden kann. Zum kontinuierlichen Betrieb kann der Trogkneter mit einem Austragzylinder und einer Austragschnecke, die entweder gegenläufig zum Troginnern arbeitet und damit den Misch- und Knetvorgang verstärkt oder die in umgekehrter Richtung zur Austragung des Mischgutes geschaltet wird, ausgestattet werden.

Zu den weiteren, für einen kontinuierlichen Betrieb geeigneten Maschineneinrichtungen gehören: Einwellenmischer wie Einschneckenextruder, Ko-Kneter, Zweiwellenmischer mit gleichläufiger oder gegenläufiger Doppelschnecke, konische Cotruder-Schnecke, Zweiwellenduchlaufkneter und kontinuierliche Mehrwellengeräte, wie z.B ein Vierschneckenextruder.

Nach dem Vermischen wird die Polymergelmasse bei einer Temperatur im Bereich von 50 - 160°C getrocknet. Man erhält ein Endprodukt, in dem die Komponente B (z.B. ein Naturpolymer) im synthetischen Polymerisat so eingebunden ist, daß die mit Wasser extrahierbaren Anteile des Endprodukts deutlich niedriger, bevorzugt 30 Gew.-% und insbesondere niedriger als 20 Gew.-%, sind als bei einer physikalischen Mischung der Mischungskomponenten A und B.

Die Einbindung der Komponente B in das synthetische Polymerisat kann durch Zusatz von verschiedenen, oben schon erwähnten Katalysatoren in einer Menge von 0,01 bis 2,0 Gew.-% und/oder oben bereits erwähnten mehrfunktionellen Monomeren in einer Menge von 0,05 bis 5,0 Gew.-% zu der Komponente B oder Komponente A verstärkt werden. Die Katalysatoren oder mehrfunktionellen Verbindungen können z.B. als Lösung auf die Komponenten A oder B vor oder während der Abmischung aufgesprüht werden.

Als Bindemittel für die beiden Komponenten A und B kann man noch zusätzlich als Hilfsmittel zur Bindung der beiden Komponenten die nieder- oder hochmolekularen, wasserlöslichen oder wasserquellbaren Polymerisate auf synthetischer oder natürlicher Grundlage (z.B. Polysaccharide in gelöstem oder aufgequollenem Zustand) verwenden. Beispiele für wasserlösliche oder wasserquellbare Polymerisate auf synthetischer Basis sind die Polymeren oder Copolymeren auf der Basis von (Meth-)Acrylsäure oder (Meth-)Acrylsäurederivaten wie die Homo- oder Copolymere der Acryl-, Methacryl-. 2-Acrylamido-2-methylpropansulfonsäure, der Salze dieser Säuren, des Acryl- oder Methacrylamids untereinander oder mit Vinylpyrrolidon und/oder Vinylacetat sowie Polyvinylalkohol.

Es können aber auch nieder- oder hochmolekulare Polymerisate, die als Emulsionspolymerisate in einer wässrigen Dispersion in Form der durch Emulgator solubilisierten winzigen kugelförmigen Partikel vorhanden sind, verwendet werden, wobei beide Emulsionsformen "Öl-in-Wasser" (für wasserunlösliche Polymere) sowie "Wasser-in-Öl" (für wasserlösliche Polymere) möglich sind. Die Ölphase besteht bekanntlich meist aus mit Wasser nicht mischbaren organischen Lösungsmitteln wie aliphatischen oder aromatischen Kohlenwasserstoffen (z.B. Hexan, Weißöl).

Beispiele für Polymerisate, die Öl-in-Wasser-Emulsionen bilden können, sind Polymerisate von Butadien, Styrol, Isopren, Chloropren, Acrylnitril, Vinylacetat, Vinyl- und Vinylidenchlorid, Alkylacrylate und -methacrylate und Copolymerisate von diesen Monomeren untereinander sowie auch mit Methylstyrol, Isobutylen oder Äthylen.

Als Beispiele für Polymerisate, die in Wasser-in-Öl-Emulsionen verwendet werden, sind die schon o.a. wasserlöslichen oder wasserquellbaren Polymerisate bzw. Copolymerisate auf Basis von (Meth)(Meth)-Acrylsäurederivaten, die unvernetzt oder auch vernetzt sein können.

Die nach dem erfindungsgemäßen Verfahren aus Komponenten A und B hergestellten Endprodukte zeigen, wie in den nachfolgenden Beispielen dargestellt ist, ein verbessertes Aufnahmevermögen für synthetische Modellurinlösung (Beispiele 1 - 8), bei einer deutlich besseren Abbaubarkeit der Endprodukte, (Beispiel 16).

Das Endprodukt besteht aus den Komponenten A und B in einem Gewichtsverhältnis 20 bis 98 Gew.-%, vorzugsweise 40 bis 95 Gew.-% der Komponente A, zu 2 bis 80 Gew.-%, vorzugsweise 5 bis 60 Gew.-%, der Komponente B.

### Prüfmethoden:

1) Zur Ermittlung der Aufnahmegeschwindigkeit wird die Aufnahme von Modellurin nach dem Demand-Absorbency-Test (DAT-Methode nach W. F. Schlauch, Vortrag Index 1978, Amsterdam) durchgeführt und die Aufnahmegeschwindigkeit nach 60 Sekunden sowie die Maximalaufnahme und Retention ermittelt. Das Meßgerät besteht aus einer Bürette, die mit der Modellurinlösung (2,0 % Harnstoff, 0,9 % NaCl, 0,1 % MgSO₄ und 0,06 % CaCl₂, aufgelöst in destilliertem Wasser) gefüllt ist, und einem Probetisch, der mit einer an die Meßbürette angeschlossenen Öffnung für den Modellurinlösungsaustritt versehen ist. Auf dem mit einem dünnen Vlies (10 x 13,5 cm) bedeckten Probetisch werden 0,5 g des erfindungsgemäßen Produktes, vermischt mit 5 mg Aerosil 200, in Form einer kreisrunden Fläche von 4,5 cm Durchmesser, zentrisch über dem Flüssigkeitsaustritt, gleichmäßig aufgestreut. Durch Schließen des Schlauches und leichte Druckgebung wird der Kontakt der Modellurinlösung mit dem Pulverprodukt hergestellt, so daß die Modellurinlösung durch das erfindungsgemäße Produkt absorbiert werden kann. Die absorbierte Menge der Modellurinlösung wird nach 20 - 30 Minuten als Maximalwert abgelesen. Anschließend wurde die Retention durch Belastung des gequollenen Geles mit einem Gewicht von 10 g/cm² ermittelt; die Zeit der Belastung betrug 5 Minuten. Die ermittelten Retentionswerte sind in den Beispielen tabellenweise zusammengefaßt.
2) Als weitere Methode zur Bestimmung der Flüssigkeitsaufnahmegeschwindigkeit wurde ein Teebeuteltest durchgeführt, wobei die Flüssigkeitsaufnahme von 0,2 g Prüfsubstanz ohne Aerosilzusatz in einem Teebeutel nach 10 Minuten (Maximalwert) und nach dem Schleudern in einer Zentrifuge, beispielsweise in einer handelsüblichen Wäscheschleuder bei 1400 Upm gravimetrisch ermittelt und auf 1 g Produkt umgerechnet wurde (Retentionswert). Als Prüfflüssigkeit wurde die wässrige 0,9%ige NaCl-Lösung verwendet.
3) Künstliche Abbaubedingungen durch dem Tageslicht ähnliche Bestrahlung wurden mit Xenotestlampe und Bestrahlungszeiten von 30, 60 und 90 Min. an mit Wasser gequollenem Polymergel (200 g Wasser auf 1 g Produkt) simuliert. Das gequollene Polymergel wurde mit der Xenotestlampe bestrahlt, und nach bestimmten Zeiten wurde die Abbaubarkeit des Polymergels nach einer Skala mit Stufen 1 bis 8 beurteilt (siehe Beispiel 16).

- Stufe 1:: Gelstruktur unverändert
- Stufe 2:: Gelstruktur leicht verändert
- Stufe 3:: Gelstruktur noch erkennbar, aber leichtes Fließen
- Stufe 4:: Gelstruktur stark verflossen
- Stufe 5:: Gelstruktur nicht mehr erkennbar, hochviskose Flüssigkeit
- Stufe 6:: leichtviskose Flüssigkeit
- Stufe 7:: wasserähnliche Flüssigkeit
- Stufe 8:: Wasser verdampfte vollständig

Die Xenotestlampe entspricht im spektralen Bereich annähernd dem Tageslicht, und die Methode wurde von den Cassella Farbwerken, Mainkur AG, Frankfurt, entwickelt.

### Beispiele 1 bis 3:

In einem Polymerisationsgefäß wurden 330 g Acrylsäure, 2,6 g N,N'-Methylenbisacrylamid (0,8 Gew.-%, bezogen auf Acrylsäure) in 1000 ml Wasser gelöst und mit 130 g Natriumhydrogencarbonat teilneutralisiert. Bei Raumtemperatur wurden die Katalysatorkomponenten (0,3 g Azobisamidinpropandihydrochlorid, 0,6 g Kaliumperoxidisulfat und 1,2 g Natriumpyrosulfit), gelöst in 120 ml Wasser, zugegeben und die adiabatische Polymerisation gestartet. Das bereits gebildete Polymergel wurde zerkleinert, mit einer wässrigen Lösung von 2000 ppm (bezogen auf Trockensubstanz des Polymergels) Natriumperoxidisulfat gleichmäßig besprüht und mit Komponente B vermischt.

Die Vermischung des Polymergels A mit der Komponente B erfolgte in einem Drais-Mischer mit rotierendem Mischwerkzeug. Das Gerät besteht aus einem vertikal oder horizontal gelegenen Metallzylinder (Volumen ca. 6000 ml), dessen Mischwerkzeug mit Leitschaufeln, die die Wände des Mischerzylinders gründlich abstreifen, ausgerüstet ist. Nachdem eine Rührwerksdrehzahl von 300 Upm erreicht wurde, bildet sich beim Austreten der rotierenden Leitschaufeln des Mischwerkzeuges aus den herausgeschleuderten einzelnen Teilchen der Gutmasse ein Fließbett auf der ganzen Länge des Metallzylinders. In diesem Stadium erfolgt eine gleichmäßige Durchmischung und Durchknetung der beiden Komponenten.

Anschließend wurde die entstandene Polymergelmasse bei 120°C im Umlufttrockenschrank getrocknet und danach gemahlen. Als Komponente B wurde kaltwasserlösliche Stärke in verschiedenen Verhältnissen verwendet. Das Aufnahmevermögen der Endprodukte wurde nach der DAT-Methode ermittelt (s. Tabelle 1).

**Tabelle 1:**

| | Komponente A | Komponente B | D A T - Werte | |
|---|---|---|---|---|
| | Acrylsäurepolymerisat Gew.-% | kaltwasserlösl. Stärke Gew.-% | Retention | |
| | | | (a) (ml/g) | (b) (ml/g) |
| | 100 | 0 | 26,9 | 26.9 |
| | 0 | 100 | kl.als 1,0 | -- |
| Beispiel 1 | 91 | 9 - | 26,0 | 28,6 |
| Beispiel 2 | 83 | 17 | 23,7 | 28,5 |
| Beispiel 3 | 67 | 33 | 20,0 | 29,8 |
| Bemerkung: Der DAT-Wert Retention (a) bezieht sich auf 1 g des zusammengesetzten Produktes aus Komponenten A und B, die Retention (b) bezieht sich auf 1 g der Komponente A im Produkt. | | | | |

### Beispiele 4 bis 6:

Nach dem Verfahren gemäß Beispielen 1 bis 3 wurde ein Polymergel mit einem Vernetzergehalt von 0,6 Gew.-% (bezogen auf Polyacrylsäure) hergestellt und als Komponente A mit unterschiedlichen Mengen an Maisstärke im bereits beschriebenen Mischer vermischt und in gleicher Weise, wie in den Beispielen 1 bis 3 beschrieben, verarbeitet.

**Tabelle 2:**

| | Komponente A | Komponente B | Teebeuteltest | |
|---|---|---|---|---|
| | Acrylsäurepolymerisat Gew.-% | Maisstärke Gew.-% | Retention | |
| | | | (a) (ml/g) | (b) (ml/g) |
| | 100 | 0 | 26,9 | 26,9 |
| | 0 | 100 | kl. als 1,0 | -- |
| Beispiel 4 | 91 | 9 | 26,0 | 28,2 |
| Beispiel 5 | 83 | 17 | 25,1 | 30,2 |
| Beispiel 6 | 71 | 29 | 24,8 | 34,9 |
| Bemerkung: Die Teebeuteltest-Retention (a) bezieht sich auf 1 g des zusammengesetzten Produktes aus Komponenten A und B, die Retention (b) bezieht sich auf 1 g der Komponente A im Produkt. | | | | |

### Beispiele 7 und 8:

In einem Polymerisationsgefäß wurden 370 g Acrylsäure, 3,1 g N,N'-Methylenbisacrylamid in 410 ml Wasser gelöst und mit 460 g Natronlauge (45%ig) neutralisiert. Bei Raumtemperatur wurden die Katalysatorkomponenten (0,26 g Azobisamidinpropandihydrochlorid, 0,06 g Benzildimethylketal (Irgacure 651) und 0,26 g t-Butylhydroperoxid, gelöst in Wasser, zugegeben und die adiabatische Polymerisation durch UV-Licht gestartet. Der erreichte Umsatz betrug 99,5 %. Das entstandene Polymergel wurde zerkleinert, bis auf einen Wassergehalt von 8 Gew.-% getrocknet, gemahlen und in einer Mischeinrichtung gemäß den Beispielen 1 - 3 mit Maisstärke und angefeuchteten Viskosekurzschnittfasern vermischt und bei 120°C nochmals getrocknet. Die Ergebnisse sind in nachfolgender Tabelle zusammengefaßt.

**Tabelle 3:**

| | Komponente A | Komponente B | | Teebeuteltest-Werte | | |
|---|---|---|---|---|---|---|
| | Acrylsäurepolymerisat Gew.-% | Maisstärke Gew.-% | Viskosefasern Gew.-% | Maxim al (ml/g) | Retention | |
| | | | | | (a) (ml/g) | (b) (ml/g) |
| | 100 | 0 | 0 | 39,8 | 24,2 | 24,2 |
| | 0 | 100 | 0 | 4,0 | kl.1,0 | - |
| | 0 | 0 | 100 | 5,8 | 1,0 | - |
| Beispiel 7 | 80 | 5 | 15 | 37,1 | 23,8 | 29,7 |
| Beispiel 8 | 50 | 5 | 45 | 31,5 | 15,4 | 30,8 |
| Bemerkung: Die Teebeuteltest-Werte Maximal und Retention (a) beziehen sich auf 1 g des zusammengesetzten Produktes aus Komponenten A und B, die Retention (b) bezieht sich auf 1 g der Komponente A im Produkt. | | | | | | |

### Beispiele 9 und 10:

Nach dem Verfahren gemäß den Beispielen 1 bis 3 wurde ein Polymergel mit einem Vernetzergehalt von 0,4 Gew.-% (bezogen auf Polyacrylsäure) hergestellt und als Komponente A mit Maisstärke und unterschiedlichen Mengen an Polyamidkurzfasern (1,0 mm, 6,7 dtex) in einem Trogkneter vermischt und verarbeitet. Der benutzte Trogkneter (Werner & Pfleiderer, Stuttgart) bestand aus einem Gehäuse mit Doppel-U-Querschnitt, in dem sich ein Knetwellenpaar mit zwei gezähnten, gegensinnig laufenden Sigmaschaufeln bewegte. Die Bearbeitung im Kneter wurde nach 5 Minuten beendet, die entstandene Knetmasse im Fleischwolf zerkleinert und bei 120°C getrocknet. Die Zusammensetzung und die Teebeuteltestwerte sind in nachfolgender Tabelle 4 aufgeführt.

**Tabelle 4:**

| | Komponente A | Komponente B | | Teebeuteltest-Werte | | |
|---|---|---|---|---|---|---|
| | Acrylsäurepolymerisat Gew.-% | Maisstärke Gew.-% | Polyamidfasern Gew.-% | Maximal (ml/g) | Retention | |
| | | | | | (a) (ml/g) | (b) (ml/g) |
| | 100 | 0 | 0 | 39,8 | 30,2 | 30,2 |
| | 0 | 100 | 0 | 4,0 | kl.a.1,0 | - |
| | 0 | 0 | 100 | 4,5 | kl.a.1,0 | - |
| Beispiel 9 | 90 | 5 | 5 | 39,4 | 27,5 | 30,6 |
| Beispiel 10 | 85 | 5 | 10 | 39,8 | 27,4 | 32,2 |
| Bemerkung: Die Teebeuteltest-Werte Maximal und Retention (a) beziehen sich auf 1 g des zusammengesetzten Produktes aus Komponenten A und B, die Retention (b) bezieht sich auf 1 g der Komponente A im Produkt. | | | | | | |

### Beispiel 11:

Nach dem Verfahren gemäß den Beispielen 1 bis 3 wurde ein Polymergel mit einem Vernetzergehalt von 0,6 Gew.-% (bezogen auf Polyacrylsäure) hergestellt und als Komponente A mit unterschiedlichen Mengen an Maisstärke in einem Mischer nach den Beispielen 1 - 3 vermischt und in gleicher Weise, wie in den Beispielen 1 bis 4 beschrieben, verarbeitet. Das pulverförmige Endprodukt wurde mit Wasser gequollen (200 g Wasser auf 1 g Produkt) und das gequollene Gel wurde mit Xenotestlampe bestrahlt, um künstliche Abbaubedingungen zu simulieren. Nach 30, 60 und 90 Minuten wurde die Abbaubarkeit des Polymergels nach einer Skala mit Stufen 1 bis 8 beurteilt. Die Ergebnisse sind in nachfolgender Tabelle zusammengefaßt.

**Tabelle 5:**

| Komponente A | Komponente B | Belichtungszeit | | |
|---|---|---|---|---|
| Acrylsäurepolymerisat Gew.-% | Maisstärke Gew.-% 30 | Minuten | | |
| | | 30 | 60 | 90 |
| 100 | 0 | 1 | 1 | 2 |
| 90 | 10 | 2 | 4 | 5 |
| 80 | 20 | 3 | 4 | 6 |
| 60 | 40 | 3 | 6 | 7 |

- Stufe 1:: Gelstruktur unverändert
- Stufe 2:: Gelstruktur leicht verändert
- Stufe 3:: Gelstruktur noch erkennbar, aber leichtes Fließen
- Stufe 4:: Gelstruktur stark verflossen
- Stufe 5:: Gelstruktur nicht mehr erkennbar, hochviskose Flüssigkeit
- Stufe 6:: leichtviskose Flüssigkeit
- Stufe 7:: wasserähnliche Flüssigkeit
- Stufe 8:: Wasser verdampfte vollständig

### Beispiel 12:

In dem im Beispiel 9 beschriebenen Trogkneter wurde die Polymerisation durchgeführt und während der Polymerisation die Stärke in das Polymergel eingearbeitet. In dem mit Stickstoff ausgeblasenen Kneter wurden zunächst 840 g Acrylsäure, 5,88 g Methylenbisacrylamid in 1270 ml Wasser gelöst und mit 725 g Natronlauge (45%ig) teilneutralisiert. Bei Raumtemperatur wurden die Katalysatorkomponenten (3,3 g Azobisamidinpropandihydrochlorid, 3,0 Natriumperoxidisulfat und 0,05 g Ascorbinsäure), gelöst in 150 ml Wasser, zugegeben, die Monomerlösung mit Stickstoff ausgeblasen und die adiabatische Polymerisation gestartet.

Nach Erreichen von einem Polymerisationsumsatz von ca. 60 % wurden 84 g kaltwasserlösliche Stärke (10 Gew.-%, bezogen auf Acrylsäure) mit dem Knetwellenpaar in das sich bildende weiche Polymergel portionsweise eingerührt und die Polymerisation weitergeführt. Anschließend wurde die entstandene Polymergelmasse zerkleinert, im Umlufttrockenschrank bei 120°C getrocknet und danach gemahlen.

Das Aufnahmevermögen des Endproduktes betrug (DAT-Werte) 32 ml/g - Maximalwert und 21 ml/g - Retention (a).

### Beispiel 13:

Nach dem Verfahren gemäß Beispiel 12 wurde die Polymerisation im Trogkneter gestartet, und nachdem ein Polymerisationsumsatz von ca. 30 % erreicht wurde, erfolgte eine Zugabe von 168 g kaltwasserlöslicher Stärke (20 Gew.-%, bezogen auf Acrylsäure) zu dem sich bildenden weichen Polymergel. Mit dem Knetwellenpaar wurde die Stärke in das Polymergel eingearbeitet. Nach der beendeten Polymerisation wurde die Polymergelmasse zerkleinert und getrocknet wie im Beispiel 12.

Das Aufnahmevermögen des Endproduktes betrug 28 ml/g (DAT-Maximal) und 20 ml/g - Retention (a).

## Patentansprüche

1. Verfahren zur Herstellung von Absorptionsmaterial für Wasser, wäßrige Lösungen und Körperflüssigkeiten aus wenigstens zwei Komponenten A und B, wobei die Komponente A wenigstens ein wasserquellbares, vernetztes, synthetisches Polymeres oder Copolymeres, dessen Polymerisation zusammen mit di- oder mehrfunktionellen Monomeren als vernetzende Monomere erfolgt ist, und die Komponente B wenigstens eine natürliche polymere Verbindung ist, die bei normaler Temperatur als rieselfähiges Pulver vorliegt und in Wasser nur begrenzt löslich oder unlöslich ist, dadurch gekennzeichnet, daß als Komponente B Stärke, Stärkederivate oder modifizierte Stärke, als Pulver im trockenen bzw. im leicht angefeuchteten bis gequollenen Zustand während der Herstellung von A nach einem Polymerumsatz von mindestens 30 % dem Polymergel zugesetzt, mit diesem vermischt und anschließend getrocknet wird.

2. Verfahren nach Anspruch 1, dadurch gekennzeichnet, daß die Komponente B zugesetzt wird, nachdem ein Polymerumsatz von mindestens 60 % erreicht worden ist.

3. Verfahren nach Anspruch 1, dadurch gekennzeichnet, daß die Komponente B zugesetzt wird, nachdem ein Polymerumsatz von mehr als 80 %, bevorzugt mehr als 95 %, erreicht worden ist.

4. Verfahren nach einem der Ansprüche 1 bis 3, dadurch gekennzeichnet, daß nach beendeter Polymerisation der Komponente A getrocknet wird.

5. Verfahren nach einem der Ansprüche 1 bis 4, dadurch gekennzeichnet, daß eine Bindung zwischen den Komponenten A und B durch Zusatz wenigstens eines Radikale bildenden Katalysators und/oder mehrfunktionellen Monomeren zur Komponente A oder B erreicht wird.

6. Verfahren nach einem der Ansprüche 1 bis 5, dadurch gekennzeichnet, daß als Komponente A ein vernetztes Polymeres oder Copolymeres auf Basis von Acrylsäure, Methacrylsäure, Derivaten dieser Carbonsäuren, vorzugsweise ein Homo oder Copolymerisat der Acryl-, Methacryl-, 2-Acrylamido-2-methylpropansulfonsäure, den Alkali- oder Ammoniumsalzen dieser Carbonsäuren, des Acryl- oder Methacrylamids und deren Derivaten, des Vinylpyrrolidons sowie deren Copolymerisaten untereinander oder mit anderen nur teilweise wasserlöslichen Monomeren verwendet wird.

7. Verfahren nach Anspruch 1-6 dadurch gekennzeichnet, daß als Stärke oder Stärkederivate Mais-, Korn-, Kartoffelstärke, Amylose, Amylopektin, Dextrin, Glykogen verwendet wird.

8. Verfahren nach einem der Ansprüche 1 bis 7, dadurch gekennzeichnet, daß zusätzlich zur Komponente B ein Fasermaterial aus Naturfasern oder aus synthetischen Fasern zugesetzt wird.

9. Verfahren nach Anspruch 8, dadurch gekennzeichnet, daß als Naturfasern Wolle-, Seide-, Baumwolle-, Hanf- oder Cellulose-Fasern, vorzugsweise Viskose-, Acetat- oder Triacetatfasern, und als synthetische Fasern Polyester-, Polyolefin-, Polyamid-, Polyvinylalkohol-, Polyurethan-, Polyharnstoff- oder Polyacrylnitrilfaser verwendet werden.

10. Verfahren nach einem der Ansprüche 1 bis 7, dadurch gekennzeichnet, daß im Gemisch mit der Komponente B ein neutraler Füllstoff zugesetzt wird.

11. Verfahren nach Anspruch 10, dadurch gekennzeichnet, daß als neutraler Füllstoff Torf, Sand, Tonerde, Erde für Pflanzenzucht, gemahlene Nußschalen oder Kernobstschalen, Holzmehl und/oder chitinhaltiges Mehl verwendet wird.

12. Absorptionsmaterial erhältlich nach einem der Ansprüche 1-11.

13. Absorptionsmaterial nach Anspruch 12, dadurch gekennzeichnet, daß es aus 20-98 Gew.-%, vorzugsweise 40-95 Gew.-% der komponente A und 2-80 Gew.-%, vozugsweise 5-60 Gew.-% der komponente B besteht.

14. Verwendung des Absorptionsmaterials nach Anspruch 12 oder 13 zur Aufnahme und/oder Zurückhaltung von Wasser und/oder wäßrigen Lösungen, bevorzugt von wäßrigen Körperflüssigkeiten wie Urin oder Blut, in absorbierenden Wegwerferzeugnissen für hygienische, chirurgische und andere medizinische Zwecke wie Babywindeln, Tampons und Damenbinden.

15. Verwendung des Absorptionsmaterials nach Anspruch 12 oder 13 zur Aufnahme und/oder Zurückhaltung von Wasser und/oder wäßrigen Lösungen und zur nachfolgenden gesteuerten Abgabe von Wasser und/oder in wäßrigem Medium gelösten Substanzen an andere Körper.

## Claims

1. A process for producing an absorbent material for water, aqueous solutions and body fluids, made up of at least two components A and B, component A being at least one water-swellable, crosslinked synthetic polymer or copolymer, the polymerization of which has been effected together with di- or polyfunctional monomers as crosslinking monomers, and component B being at least one natural polymer compound which is present as a flowable powder at normal temperature and is only sparingly soluble or insoluble in water, characterized in that starch, starch derivatives, or modified starch in the form of powders in a dry or from slightly wetted to swollen state are added as component B to the polymer gel during the production of A after a polymer conversion of at least 30%, mixed with said polymer gel and subsequently dried.

2. The process according to claim 1, characterized in that component B is added after a polymer conversion of at least 60% has been reached.

3. The process according to claim 1, characterized in that component B is added after a polymer conversion of more than 80%, preferably more than 95% has been reached.

4. The process according to any of claims 1 to 3, characterized in that drying is effected after completed polymerization of component A.

5. The process according to any of claims 1 to 4, characterized in that bonding between components A and B is achieved by adding at least one free radical-forming catalyst and/or polyfunctional monomers to component A or B.

6. The process according to any of claims 1 to 5, characterized in that a crosslinked polymer or copolymer based on acrylic acid, methacrylic acid, derivatives of these carboxylic acids, preferably a homo- or copolymer of acrylic, methacrylic, 2-acrylamido-2-methylpropanesulfonic acid, the alkali or ammonium salts of these carboxylic acids, of acryl- or methacrylamide and derivatives thereof, of vinylpyrrolidone, as well as copolymers between each of these or with other, only partially water-soluble monomers are used as component A.

7. The process according to any of claims 1 to 6, characterized in that corn, cereal, potato starches, amylose, amylopectin, dextrin, glycogen are used as starch or starch derivatives.

8. The process according to any of claims 1 to 7, characterized in that a fibrous material made of natural fibers or synthetic fibers is additionally added to component B.

9. The process according to claim 8, characterized in that wool, silk, cotton, hemp, or cellulose fibers, preferably viscose, acetate or triacetate fibers are used as natural fibers, and polyester, polyolefine, polyamide, polyvinyl alcohol, polyurethane, polyurea, or polyacrylonitrile fibers are used as synthetic fibers.

10. The process according to any of claims 1 to 7, characterized in that a neutral filler is added in admixture with component B.

11. The process according to claim 10, characterized in that peat, sand, clay, plant breeding soil, ground nutshells, or pome shells, wood meal and/or chitinous meal are used as neutral filler.

12. An absorbent material which can be obtained according to any of claims 1 to 11.

13. The absorbent material according to claim 12, characterized in that the material is comprised of 20-98 wt.-%, preferably 40-95 wt.-% of component A, and 2-80 wt.-%, preferably 5-60 wt.-% of component B.

14. Use of the absorbent material according to claim 12 or 13 for absorbing and/or retaining water and/or aqueous solutions, preferably aqueous body fluids such as urine or blood, in absorbent disposable articles for hygienic, surgical and medical purposes, such as diapers for babies, tampons and sanitary towels.

15. Use of the absorbent material according to claim 12 or 13 for absorbing and/or retaining water and/or aqueous solutions, and in the subsequent controlled release of water and/or substances dissolved in the aqueous medium to other bodies.

## Revendications

1. Procédé de fabrication d'un matériau absorbant l'eau, les solutions aqueuses et les liquides corporels, constitué d'au moins deux composants A et B, dans lequel le composant A est au moins un polymère ou un copolymère synthétique, réticulé, gonflable à l'eau, dont la polymérisation s'effectue ensemble avec des monomères bifonctionnels ou plurifonctionnels faisant office de monomères réticulants, et dans lequel le composant B est au moins un composé polymère naturel, qui est, à température normale, sous forme de poudre coulante et qui n'est soluble ou insoluble dans l'eau que d'une manière limitée, caractérisé en ce qu'en tant que composant B, de l'amidon, un dérivé de l'amidon ou de l'amidon modifié est ajouté sous forme de poudre sèche ou légèrement humidifiée à gonflée au cours de la fabrication de A au gel de polymère après un taux de conversion en polymère de 30 %, qu'il est mélangé avec celui-ci et qu'il est ensuite séché.

2. Procédé d'après la revendication 1, caractérisé en ce que le composant B est ajouté après qu'un taux de conversion en polymère d'au moins 60 % a été atteint.

3. Procédé d'après la revendication 1, caractérisé en ce que le composant B est ajouté après qu'un taux de conversion en polymère supérieur à 80 %, de préférence supérieur à 95 % a été atteint.

4. Procédé d'après une des revendications de 1 à 3, caractérisé en ce qu'après la polymérisation terminée, le composant A est séché.

5. Procédé d'après une des revendications de 1 à 4, caractérisé en ce qu'une liaison des composants A et B est obtenue par ajout d'au moins un catalyseur générant des radicaux libres et/ou de monomères plurifonctionnels aux composants A ou B.

6. Procédé d'après une des revendications de 1 à 5, caractérisé en ce qu'en tant que composant A, l'on utilise un polymère ou un copolymère réticulé, à base d'acide acrylique, d'acide méthacrylique, de dérivés de ces acides carboxyliques, de préférence un homo- ou copolymère d'acide acrylique, d'acide méthacrylique, d'acide 2-acrylamido-2-méthylpropanesulfonique, du sel alcalin ou du sel d'ammonium de ces acides carboxyliques, d'acryl- ou de méthacrylamide et des dérivés, de vinylpyrrolidone, ainsi qu'un de leurs copolymères obtenu par mélange d'entre eux ou par mélange avec d'autres monomères solubles dans l'eau seulement en partie.

7. Procédé d'après une des revendications de 1 à 6, caractérisé en ce qu'en tant que l'amidon ou les dérivés d'amidon, l'on utilise de l'amidon de maïs, de l'amidon de blé, de l'amidon de pomme de terre, de l'amylose, de l'amylopectine, de la dextrine ou du glycogène.

8. Procédé d'après une des revendications de 1 à 7, caractérisé en ce qu'en plus du composant B, un matériau fibreux en fibre naturelle ou en fibre synthétique est ajouté.

9. Procédé d'après la revendication 8 caractérisé en ce qu'en tant que fibres naturelles, l'on utilise des fibres de laine, de soie, de coton, de chanvre ou de cellulose, de préférence, des fibres de viscose, d'acétate ou de triacétate, et qu'en tant que fibres synthétiques, l'on utilise de la fibre de polyester, de polyoléfine, de polyvinylalcool, de polyuréthane, de polyurée ou de polyacrylonitrile.

10. Procédé d'après une des revendications de 1 à 7, caractérisé en ce qu'une charge neutre est ajoutée dans le mélange avec le composant B.

11. Procédé d'après la revendication10, caractérisé en ce qu'en tant que la charge neutre, l'on utilise de la tourbe, du sable, de l'alumine, de la terre pour la culture des plantes, des coquilles de noix ou de fruits à noyaux concassées, de la farine de bois et/ou de la farine contenant de la chitine.

12. Matériau absorbant obtenu d'après une des revendications de 1 à 11.

13. Matériau absorbant d'après la revendication 12, caractérisé en ce qu'il est composé de 20 à 98 % en poids, de préférence de 40 à 95 % en poids du composant A et de 2 à 80 % en poids, de préférence de 5 à 60 % du composant B.

14. Utilisation du matériau absorbant d'après les revendications 12 ou 13 pour l'absorption et/ou la rétention d'eau et/ou de solutions aqueuses, en particulier de liquides corporels aqueux comme l'urine ou le sang, dans des produits absorbants jetables pour des applications d'hygiène, de chirurgie ou d'autres applications médicales comme les langes de bébé, les tampons et les serviettes hygiéniques.

15. Utilisation du matériau absorbant d'après les revendications 12 ou 13 pour l'absorption et/ou la rétention d'eau et/ou de solutions aqueuses et pour la cession suivante contrôlée à d'autres corps d'eau et/ou de substances dissoutes dans un milieu aqueux.
